# FASCICULE DE BREVET EUROPEEN

(11) **EP 0 780 155 B1**
(45) Date de publication et mention de la délivrance du brevet: **26.09.2001**
(21) Numéro de dépôt: 96402687.6
(22) Date de dépôt: 10.12.1996
(51) Int. Cl.: B01J 23/50, B01J 23/44, C07C 5/09, B01J 23/58

(54) **Catalyseur d'hydrogénation sélective et procédé utilisant ce catalyseur**
Selektive Hydrierungskatalysator und Verfahren unter Verwendung derselben
Selective hydrogenation catalyst and process using this catalyst

(30) Priorité: 22.12.1995 FR 9515340
(43) Date de publication de la demande: 25.06.1997
(73) Titulaire: INSTITUT FRANCAIS DU PETROLE, 92502 Rueil-Malmaison (FR)
(72) Inventeur: Didillon, Blaise, 92500 Rueil Malmaison (FR); Nguyen Thanh, Canh, 78170 La Celle Saint Cloud (FR); Sarrazin, Patrick, 92500 Rueil Malmaison (FR); Cameron, Charles, 75005 Paris (FR)

(56) Documents cités:
- EP-A- 0 064 301
- EP-A- 0 686 615
- US-A- 3 651 167
- US-A- 5 004 859

## Description

L'invention concerne un nouveau catalyseur supporté utilisable notamment dans l'hydrogénation sélective en phase gazeuse des hydrocarbures acétyléniques de 2 ou 3 atomes de carbone en les hydrocarbures éthyléniques correspondants. Elle concerne plus particulièrement un catalyseur pour l'hydrogénation sélective de composés acétyléniques tels que l'acétylène ou le propyne, respectivement en éthylène ou en propylène en phase gazeuse.

Elle concerne également un procédé d'hydrogénation sélective utilisant ce catalyseur.

L'éthylène est un monomère utilisé pour la préparation d'un grand nombre de polymères. Il est généralement obtenu par des procédés de pyrolyse ou de vapocraquage d'hydrocarbures. L'éthylène ainsi produit contient de faibles quantités d'acétylène (généralement inférieures à 3 %), qu'il est nécessaire d'éliminer avant utilisation. Les teneurs en acétylène dans l'éthylène généralement tolérées pour son utilisation pour la fabrication de polymères sont généralement inférieures à 10 ppm et le plus souvent inférieures à 5 ppm.

Une des techniques utilisées pour éliminer l'acétylène dans l'éthylène est de l'hydrogéner sélectivement en éthylène en présence d'un catalyseur à base de palladium supporté sur un support réfractaire tel que l'alumine. Le problème généralement rencontré avec les catalyseurs monométalliques (constitués uniquement de palladium supporté sur alumine) est que, lorsque les conditions opératoires sont amenées pour permettre l'élimination totale de l'acétylène, une partie de l'éthylène est aussi convertie en éthane. De plus, ces catalyseurs monométalliques présentent généralement des stabilités relativement faibles du fait de la formation importante d'oligomères qui recouvrent progressivement la surface du catalyseur dans les conditions réactionnelles. Ce dépôt hydrocarboné peut certes être éliminé par des procédés d'oxydation ménagée, mais il est avantageux, dans un procédé industriel, d'avoir une durée de fonctionnement du catalyseur entre deux régénérations la plus importante possible.

Pour améliorer les propriétés des catalyseurs, l'ajout au palladium de promoteurs a depuis longtemps été décrit. Ces ajouts peuvent être par exemple l'argent (brevet US-A-2 802 889) ou le fer et l'argent (brevet US-A-3 243 387).

Ces promoteurs peuvent également être choisis parmi les métaux alcalins ou alcalino-terreux tels que le lithium (brevet US-A-3 325 556), le potassium (demande EP-A-124744) ou le calcium (brevet US-A- 4 329 530).

Que ce soit pour les catalyseurs monométalliques (catalyseurs à base de palladium uniquement) ou les catalyseurs promus (catalyseurs comprenant du palladium et au moins un autre élément), il est connu par l'homme du métier que lorsque le palladium est concentré à la surface des particules (par exemple billes) dudit catalyseur, ses performances catalytiques sont nettement supérieures à celles d'un catalyseur de formule identique pour lequel le palladium est réparti de façon homogène dans les particules de catalyseur. Par exemple dans le cas de l'utilisation des formules bimétalliques palladium-argent, il a été découvert que lorsque le palladium était situé à la périphérie des billes du catalyseur et que l'argent y était réparti de façon homogène, ceci conférait audit catalyseur de meilleures propriétés (Brevets US-A-4 404 124; EP-0064301 et FR-A-2597113), notamment la formation moins importante d'éthane et de produits d'oligomérisation.

On connaît par ailleurs la demande de brevet japonaise JP-A-04 108540 qui décrit des catalyseurs d'hydrogénation sélective en phase liquide du butadiène 1-3, dans lesquels de l'argent est précipité et supporté à la surface du palladium. Dans ces catalyseurs, le support consiste en de l'alumine de surface spécifique relativement élevée et le rapport pondéral Ag/Pd est de 0,3 à 5,0 de préférence de 0,5 à 3,0.

La demanderesse a décrit dans une demande de brevet antérieure (FR-A-2721018 déposée le 9 juin 1994) un procédé d'hydrogénation sélective en phase gazeuse des hydrocarbures acétyléniques de 2 ou 3 atomes de carbones (acétylène ou propyne) en les hydrocarbures éthyléniques correspondants (éthylène ou propylène) utilisant un catalyseur sous forme de billes ou d'extrudés contenant du palladium, au moins un métal du groupe IB de la classification périodique et de l'alumine, dans lequel une proportion d'au moins 80 % du palladium et une proportion d'au moins 80 % du métal du groupe IB étaient présentes dans un volume à la périphérie du catalyseur délimité par une surface sphérique ou cylindrique de rayon r₁ correspondant au rayon moyen des billes ou des extrudés de catalyseur et une surface sphérique ou cylindrique de rayon r₂ au moins égal à 0,8 r₁. Le rapport pondéral argent/palladium était compris entre 0,05 et 0,4, de préférence entre 0,05 et 0,25.

Plus particulièrement, la teneur en palladium était comprise entre 0,01 et 0,05 % en poids du catalyseur. L'élément du groupe IB, le plus souvent l'argent, était présent sous une teneur comprise entre 0,001 et 0,02% en poids du catalyseur.

Dans l'application industrielle envisagée, il est nécessaire d'avoir un catalyseur dont les performances varient le moins possible en fonction du temps. En effet, un catalyseur pour lequel l'acétylène est dans un premier temps totalement converti, mais pour lequel l'acétylène apparaîtrait très rapidement à des concentrations élevées en sortie de réacteur, ne pourrait être utilisé car il engendrait des problèmes évidents de pilotage de l'unité industrielle. Ceci résulte en ce qu'un des critères de choix du catalyseur d'hydrogénation est sa désactivation, qui correspond à la vitesse d'apparition de l'acétylène en sortie de réacteur. Cette désactivation devra être la plus faible possible.

On a maintenant découvert qu'il était possible de réduire la tendance à la désactivation de catalyseurs d'hydrogénation de l'acétylène tels que définis par la demandresse dans la demande de brevet FR-A-2721018. déposé le 6 juin 1994.

A cet effet, l'invention propose un nouveau catalyseur sous forme de billes ou d'extrudés subtantiellement cylindriques défini d'une manière générale en ce qu'il comprend du palladium, de l'argent et un support d'alumine, une proportion d'au moins 80 % du palladium et une proportion d'au moins 80 % de l'argent étant présentes dans un volume à la périphérie du catalyseur délimité par une surface sphérique ou cylindrique de rayon r1 correspondant au rayon moyen des billes ou des extrudés de catalyseur et une surface sphérique ou cylindrique de rayon r2 au moins égal à 0,8 r1, ledit catalyseur étant caractérisé en ce que le rapport pondéral de l'argent au palladium est de 0,4 à 3 et en ce que au moins 30 % des particules métalliques sur le support d'alumine contiennent à la fois du palladium et de l'argent.

Dans le cas de catalyseurs sous forme de billes ou d'extrudés, r₁ et r₂ peuvent être représentés comme suit :

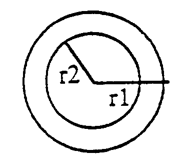

De manière préférée, le rapport pondéral de l'argent au palladium est de 0,5 à 3 et de manière encore plus préférée, il est de 0,5 à 2,5.

Le nombre de particules metalliques sur le support d'alumine est déterminé par microscopie électronique. De manière préférée au moins 40 % et de manière encore plus préférée au moins 50 % des particules métalliques contiennent à la fois du palladium et de l'argent.

Plus particulièrement, la teneur en palladium du catalyseur est comprise entre 0,01 et 0,5 % en poids et sa teneur en argent est comprise entre 0,001 et 0,1 % en poids.

Le support utilisé est une alumine et plus particulièrement cette alumine est essentiellement constituée d'aluminium alpha. D'une façon courante, il est utilisé sous la forme de billes ou d'extrudés de diamètres généralement compris entre 2 et 4 mm. Les caractéristiques de l'alumine utilisée sont généralement les suivantes: une surface spécifique comprise entre 5 et 60 m²/g et de préférence entre 5 et 15 m²/g ; un volume poreux de 0,3 à 0,95 cm³/g et un diamètre de pores supérieur à 100 Å. Ces différentes caractéristiques sont déterminées par les techniques d'analyse connues par l'homme du métier.

Le palladium peut être introduit selon les techniques connues par l'homme du métier permettant d'obtenir une répartition du palladium à la surface des billes ou des extrudés de support, qui correspond aux critères décrits plus haut. La bonne répartition du palladium peut être vérifiée par les techniques classiques telles que par exemple la microsonde de Castaing. Le palladium peut par exemple être introduit par des techniques d'imprégnation de solution aqueuse ou organique d'un précurseur de palladium. Ce précurseur peut par exemple être un composé minéral tel que le chlorure de palladium, le nitrate de palladium, le palladium tétrammine dihydroxyde, le chlorure de palladium tétrammine, ou un composé organométallique, tel que par exemple le palladium bis π allyle ou le palladium bis-acétylacétonate.

L'argent est introduit de telle sorte qu'il reste concentré à la périphérie des billes ou des extrudés du support. L'analyse de la teneur en argent après abrasion contrôlée des billes de catalyseur permet de s'assurer de la bonne répartition de l'argent dans les billes ou les extrudés de catalyseur. Quand la teneur en argent est suffisante, la bonne répartition de l'argent peut être vérifiée par microsonde de Castaing. Le précurseur généralement utilisé est le nitrate d'argent. L'acétate d'argent, le citrate d'argent, le chlorure d'argent, l'oxalate d'argent peuvent par exemple aussi être utilisés. La composition des particules métalliques peut être analysée par microscopie électronique

Les catalyseurs de l'invention, utilisables dans un procédé d'hydrogénation sélective en phase gazeuse des hydrocarbures acétyléniques de 2 ou 3 atomes de carbone en les hydrocarbures éthyléniques correspondants, peuvent contenir en outre au moins un métal alcalin ou alcalino-terreux.

Dans ce cas, la teneur en métal alcalin ou alcalino-terreux du catalyseur est avantageusement choisie pour que le rapport atomique métal alcalin ou alcalino-terreux sur palladium soit compris entre 2 et 20 et de préférence, entre 4 et 15. De préférence cette teneur est comprise entre 0,05 % et 0,2 % poids du catalyseur.

A titre de métal alcalin, on met en jeu de préférence le sodium ou le potassium. A titre de métal alcalinoterreux, le magnésium est l'élément préféré.

Le métal alcalin ou alcalino-terreux est introduit selon les techniques connues par l'homme du métier. Les précurseurs généralement utilisés sont les nitrates, les acétates, les chlorures, les carbonates et les hydroxydes.

Le palladium et l'argent et éventuellement le métal alcalin ou alcalino-terreux peuvent être introduits à partir d'une solution commune de leurs précurseurs ou à partir de solutions séparées contenant chacune un ou deux éléments. Dans ce dernier cas, des traitements de séchage, de calcination ou de réduction à des températures comprises entre 120°C et 900°C peuvent éventuellement être réalisés entre deux étapes d'imprégnation consécutives.

Lorsque le palladium et l'argent sont introduits à partir de solutions différentes, les techniques de préparation qui peuvent être employées sont par exemple celles décrites dans le brevet US-A-4 533 779, qui utilise le chlorure d'argent comme précurseur ou dans le brevet US-A-4 504 593, qui utilise le citrate d'argent comme précurseur.

Le catalyseur ainsi obtenu est généralement séché à des températures comprises entre la température ambiante et 150°C. Le catalyseur ainsi séché peut être utilisé tel que ou le plus souvent il est de préférence calciné afin de décomposer les précurseurs métalliques et/ou réduit avant utilisation. La calcination est généralement réalisée en traitant ledit catalyseur sous flux d'air à une température comprise entre 400°C et 900°C. La réduction peut être réalisée par traitement du catalyseur par un gaz contenant de l'hydrogène à une température comprise entre la température ambiante et 500°C.

Le procédé d'hydrogénation de l'invention utilisant les catalyseurs décrits ci-dessus s'applique plus particulièrement à l'hydrogénation de l'acétylène présent dans un gaz contenant de l'éthylène. Afin de s'approcher des conditions réactionnelles qui permettent d'éliminer totalement l'acétylène, le rapport molaire hydrogène sur acétylène est généralement compris entre 1 et 2, la température de la réaction est généralement comprise entre 25 et 100 °C, la pression est généralement comprise entre 1 et 5 MPa. Le débit de charge exprimé en litre de charge gazeuse par litre de catalyseur et par heure est généralement compris entre 1000 et 10 000 h⁻¹.

Au cours de l'utilisation, le catalyseur se désactive du fait d'un dépôt de composés hydrocarbonés recouvrant progressivement la phase active. Lorsque les performances du catalyseur sont jugées insuffisantes, le catalyseur peut être régénéré. La régénération du catalyseur est réalisée par combustion contrôlée des espèces hydrocarbonées présentes sur celui-ci. Cette combustion est réalisée dans les conditions connues de l'homme du métier, généralement en chauffant progressivement le catalyseur en présence d'un gaz contenant de l'oxygène à une température comprise entre 350 et 500 °C.

Les exemples non limitatifs qui suivent illustrent l'invention. Les exemples 4, 5 et 7 sont donnés à titre de comparaison.

### EXEMPLE 1 (selon l'invention)

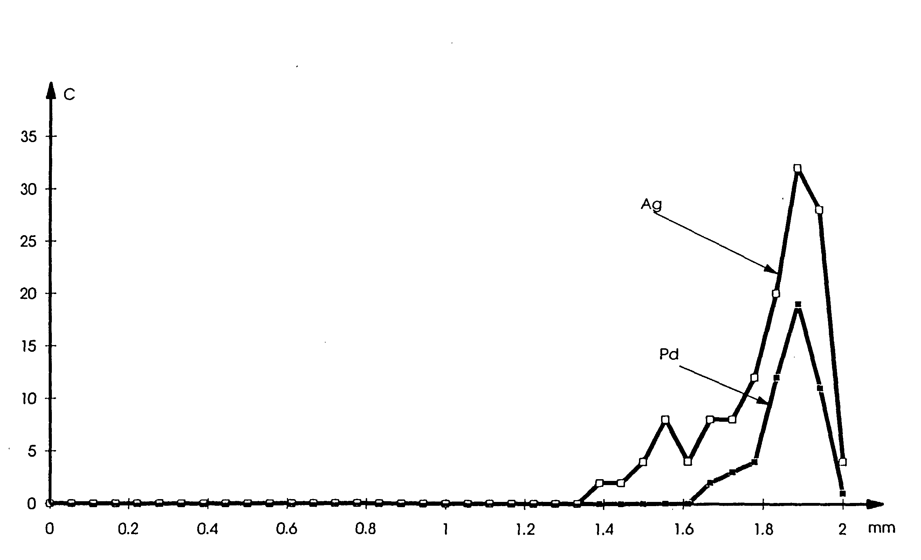

Un catalyseur selon l'invention (catalyseur A) est préparé par imprégnation de 100 g d'un support à base d'alumine alpha par 60 ml d'une solution d'acide nitrique contenant du nitrate de palladium et du nitrate d'argent. Le support utilisé se présente sous forme de billes de 2 à 4 mm de diamètres ayant une surface spécifique de 10 m²/g et un volume poreux de 0,6 cm³/g. Après imprégnation, le catalyseur est séché à 120°C et calciné sous air à 750°C. Le catalyseur A ainsi obtenu contient 0,025 % poids de palladium et 0,050 % poids d'argent. La répartition moyenne des éléments dans les grains de catalyseurs est présentée figure 1. Ces analyses montrent que plus de 80 % de l'argent et la quasi totalité du palladium sont concentrés dans un volume délimité par une sphère de rayon r1 = 2 mm et une sphère de rayon r2 de 1,6 mm. Le rapport pondéral Ag/Pd est de 2. L'analyse par microscopie électronique (STEM) des particules du catalyseur montrent que 60 % des particules contiennent à la fois du palladium et de l'argent.

### EXEMPLE 2 (selon l'invention)

Un catalyseur selon l'invention (catalyseur B) est préparé par imprégnation de 100 g d'un support à base d'alumine alpha par 60 ml d'une solution d'acide nitrique contenant du nitrate de palladium, du nitrate de sodium et du nitrate d'argent. Le support utilisé se présente sous forme de billes de 2 à 4 mm de diamètres ayant une surface spécifique de 10 m²/g et un volume poreux de 0,6 cm³/g. Après imprégnation, le catalyseur est séché à 120°C et calciné sous air à 750°C. Le catalyseur B ainsi obtenu contient 0,025 % poids de palladium, 0,05 % poids de sodium et 0,050 % poids d'argent. L'analyse par microsonde de Castaing montre une répartition des éléments à la périphérie des billes de catalyseur. Le rapport pondéral Ag/Pd est de 2. L'analyse par microscopie électronique (STEM) des particules métalliques des catalyseurs montre que 75 % des particules contiennent à la fois du palladium et de l'argent.

### EXEMPLE 3 (selon l'invention)

Un catalyseur selon l'invention (catalyseur C) est préparé par imprégnation de 100 g d'un support à base d'alumine alpha par 60 ml d'une solution d'acide nitrique contenant du nitrate de palladium, de nitrate de sodium et du nitrate d'argent. Le support utilisé se présente sous forme de billes de 2 à 4 mm de diamètres ayant une surface spécifique de 10 m²/g et un volume poreux de 0,6 cm³/g. Après imprégnation, le catalyseur est séché à 120°C et calciné sous air à 750°C. Le catalyseur C ainsi obtenu contient 0,05 % poids de palladium, 0,05 % poids de sodium et 0,052 % poids d'argent. L'analyse par microsonde de Castaing montre une répartition des éléments à la périphérie des billes de catalyseur. Le rapport pondéral Ag/Pd est de 1,04. L'analyse par miscroscopie électronique (STEM) des particules métalliques du catalyseur montre que 43 % des particules contiennent à la fois du palladium et de l'argent.

### EXEMPLE 4 (comparatif)

Un catalyseur (catalyseur D) est préparé par imprégnation de 100 g d'un support à base d'alumine alpha par 60 ml d'une solution d'acide nitrique contenant du nitrate de palladium, du nitrate de sodium, et du nitrate d'argent. Le support utilisé se présente sous forme de billes de 2 à 4 mm de diamètres ayant une surface spécifique de 10 m²/g et un volume poreux de 0,6 cm³/g. Après imprégnation, le catalyseur est séché à 120°C et calciné sous air à 750°C. Le catalyseur D ainsi obtenu contient 0,025 % poids de palladium, 0,05 % poids de sodium et 0,0025 % poids d'argent. L'analyse par microsonde de Castaing montre une répartition des éléments à la périphérie des billes de catalyseur. Mais le rapport pondéral Ag/Pd de 0,1 est en dehors de l'intervalle caractéristique de l'invention. L'analyse par microscopie électronique (STEM) montre que 35 % des particules métalliques contiennent à la fois du palladium et de l'argent.

### EXEMPLE 5 (comparatif)

Un catalyseur E comparatif est préparé en immergeant à température ambiante 100 g de support dans 120 ml d'une solution aqueuse de nitrate d'argent contenant 9 g d'argent. Le catalyseur est ainsi laissé 20 minutes sous agitation. La solution surnageante est alors éliminée. Le catalyseur est alors lavé, séché à 120°C. Sur ce solide on imprègne alors 60 ml d'une solution d'acide nitrique et de nitrate de palladium. Le catalyseur est alors séché et calciné sous air à 750°C. Le catalyseur E ainsi obtenu contient 0,025 % poids de palladium et 0,05 % d'argent. Le rapport pondéral Ag/Pd est de 2, mais l'analyse par microsonde montre que l'argent est réparti de façon homogène dans les grains de catalyseur alors que le palladium y est réparti à la périphérie. L'analyse par microscopie électronique (STEM) des particules métalliques montre que 2 % des particules analysées contiennent à la fois du palladium et de l'argent.

### EXEMPLE 6 (selon l'invention)

Un catalyseur selon l'invention (catalyseur F) est préparé par imprégnation de 100 g d'un support à base d'alumine alpha par 60 ml d'une solution d'acide nitrique contenant du nitrate de palladium, du nitrate de sodium et du nitrate d'argent. Le support utilisé se présente sous forme de billes de 2 à 4 mm de diamètres ayant une surface spécifique de 10 m2/g et un volume poreux de 0,6 cm3/g. Après imprégnation, le catalyseur est séché à 120°C et calciné sous air à 750°C. Le catalyseur F ainsi obtenu contient 0,025 % poids de palladium, 0,015 % poids de sodium et 0,030 % poids d'argent. L'analyse par microsonde de Castaing montre une répartition des éléments à la périphérie des billes de catalyseur. Le rapport pondéral Ag/Pd = 1,2. L'analyse par microscopie électronique (STEM) des particules métalliques du catalyseur montre que 40 % de celles-ci contiennent à la fois du palladium et de l'argent.

### EXEMPLE 7 (comparatif)

Un catalyseur G est préparé en imprégnant à température ambiante 100 g de support par 60 ml d'une solution de nitrate de palladium dissous dans l'acide nitrique. Le support se présente sous la forme de billes de 2 à 4 mm de diamètres ayant une surface spécifique de 10 m²/g et un volume poreux de 0,6 cm³/g. Après imprégnation, le catalyseur est séché à 120°C et calciné sous air à 750°C. Le catalyseur est ensuite imprégné par 0,6 cm³ d'une solution de nitrate d'argent. Le catalyseur est ensuite séché et calciné à 0,025 % de palladium et 0,0500 % d'argent. L'analyse par microsonde démontre que le palladium et l'argent sont répartis à la périphérie des billes de catalyseur. Le rapport Ag/Pd est de 2. L'analyse par microscopie électronique (STEM) montre que seulement 8 % des particules métalliques contiennent à la fois du palladium et de l'argent.

### EXEMPLE 8 : Comparaison des propriétés hydrogénantes des différents catalyseurs

Les tests catalytiques sont réalisés sur les catalyseurs A, B, C, D, E F et G pour déterminer leurs caractéristiques de sélectivité, de stabilité et de désactivation lors de l'hydrogénation de l'acétylène contenu dans une charge contenant 98 % d'éthylène et 2 % d'acétylène.

15 ml du catalyseur à tester sont d'abord placés dans un réacteur vertical en acier. Ce réacteur est alors placé dans un four permettant de contrôler la température. Dans un premier temps, le catalyseur est réduit sous courant d'hydrogène à 150°C pendant 2 heures sous pression atmosphérique. La température est alors portée à 50°C, le débit d'hydrogène à 1,5 l.h⁻¹ et la pression à 2,5 MPa. La charge, composée de 98 % d'éthylène et de 2 % d'acétylène, est alors injectée avec un débit volumique correspondant à une vitesse spatiale de 3300 h⁻¹. L'analyse de l'effluent gazeux en sortie de réacteur est réalisée par chromatographie en phase gazeuse. Dans ces conditions, la stabilité du catalyseur est définie comme étant le temps (en heures) à partir duquel de l'acétylène est détecté en sortie de réacteur. La sélectivité du catalyseur (en %) correspond à la teneur en éthylène de la charge après élimination totale de l'acétylène. La désactivation du catalyseur correspond à la vitesse (en % mole/heure) d'apparition de l'acétylène à partir du temps définissant la stabilité Les résultats obtenus sont rapportés dans le tableau I.

**Tableau 1.**

| Comparaison des performances des catalyseurs A, B, C, D, E, F et G pour l'hydrogénation de l'acétylène. | | | |
|---|---|---|---|
| Catalyseur | Stabilités (h) | Sélectivités (%) | Désactivation (% mole/heure) |
| A (selon l'invention) | 45 | 98,7 | 7 |
| B (selon l'invention) | 52 | 98,6 | 7 |
| C (selon l'invention) | 49 | 98,5 | 10 |
| D (comparatif) | 48 | 98,5 | 16 |
| E (comparatif) | 15 | 98,3 | 25 |
| F (selon l'invention) | 50 | 98,6 | 8 |
| G (comparatif) | 4 | 97,9 | 21 |

Ces résultats montrent clairement que lorsque le rapport Ag/Pd est de 0.6 (catalyseur F), 1,04 (catalyseur C) ou 2,0 (catalyseur A et B), la désactivation du catalyseur est inférieure à celle obtenue avec des catalyseurs où le rapport Ag/Pd = 0,1 (catalyseur D).

Quand l'argent est distribué de façon homogène dans le grain de catalyseur (catalyseur E) la pente de désactivation est plus importante et la stabilité du catalyseur est plus faible que lorsque l'argent est réparti à la périphérie des billes de catalyseur (catalyseur C). De plus, lorsque seulement 8 % des particules métalliques contiennent à la fois de l'argent et du palladium (catalyseur G), la stabilité et la sélectivité sont plus faibles et la désactivation plus importante que celles obtenues avec un catalyseur où, par exemple, 60 % des particules contiennent à la fois du palladium et de l'argent (catalyseur A).

## Revendications

1. Catalyseur sous forme de billes ou d'extrudés comprenant du palladium de l'argent et un support alumine, une proportion d'au moins 80 % du palladium et une proportion d'au moins 80 % de l'argent étant présentes dans un volume à la périphérie du catalyseur délimité par une surface sphérique ou cylindrique de rayon r1 correspondant au rayon moyen des billes ou des extrudés de catalyseur et une surface sphérique ou cylindrique de rayon r2 au moins égal à 0,8 r1, ledit catalyseur étant **caractérisé en ce que** le rapport pondéral de l'argent au palladium est de 0,4 à 3 et en ce que au moins 30 % des particules métalliques sur le support d'alumine contiennent à la fois du palladium et de l'argent.

2. Catalyseur selon la revendication 1 ou 2, **caractérisé en ce que** :
. ladite alumine a une surface spécifique de 5 à 60 m²/g,
. sa teneur en palladium est de 0,01 à 0,5 % en poids, et
. sa teneur en argent est de 0,001 à 0,1 % en poids.

3. Catalyseur selon l'une des revendications 1 et 2, **caractérisé en ce que** l'alumine a une surface spécifique de 5 à 15 m²/g.

4. Catalyseur selon l'une des revendications 1 à 3, **caractérisé en ce qu'**il comprend en outre au moins un métal alcalin ou alcalino-terreux.

5. Catalyseur selon la revendication 4, **caractérisé en ce que** le rapport atomique métal alcalin ou alcalino-terreux sur palladium est de 2 à 20.

6. ― Catalyseur selon l'une des revendications 4 et 5, **caractérisé en ce que** sa teneur en métal alcalin ou alcalino-terreux est de 0,05 à 0,2 % en poids.

7. Procédé d'hydrogénation sélective en phase gazeuse d'au moins un hydrocarbure acétylénique de 2 ou 3 atomes de carbone en l'hydrocarbure éthylénique correspondant **caractérisé en ce qu'**il comprend le passage en phase gazeuse d'une charge comprenant au moins un hydrocarbure acétylénique de 2 ou 3 atomes de carbone en présence d'hydrogène sur un catalyseur sous forme de billes ou d'extrudés selon l'une des revendications 1 à 6.

## Patentansprüche

1. Katalysator in Form von Kugeln oder Extrudaten, Palladium und Silber sowie einen Aluminiumoxidträger umfassend, wobei ein Teil von wenigstens 80% des Palladiums und ein Anteil von wenigstens 80% des Silbers in einem Volumen am Umfang des Katalysators vorhanden sind, welches durch eine Kugelfläche oder Zylinderfläche mit dem Radius r1 entsprechend dem mittleren Radius der Kugeln oder der Extrudate des Katalysators begrenzt ist und über eine kugelige oder zylindrische Oberfläche mit dem Radius r2 verfügt, die wenigstens gleich 0,8 r1 ist, wobei der Katalysator sich dadurch auszeichnet, dass das Gewichtsverhältnis des Silbers zum Palladium bei 0,4 bis 3 liegt und dass wenigstens 30% der metallischen Partikel auf dem Aluminiumoxidträger gleichzeitig Palladium und Silber enthalten.

2. Katalysator nach Anspruch 1 oder 2, **dadurch gekennzeichnet, dass**:
- dieses Aluminiumoxid eine spezifische Oberfläche von 5 bis 60 m²/g umfasst,
- sein Gehalt an Palladium zwischen 0,01 und 0,5 Gew.-% liegt und
- sein Gehalt an Silber zwischen 0,01 und 01, Gew.-% beträgt.

3. Katalysator nach einem der Ansprüche 1 oder 2, **dadurch gekennzeichnet, dass** das Aluminiumoxid eine spezifische Oberfläche von 5 bis 15 m²/g besitzt.

4. Katalysator nach einem der Ansprüche 1 bis 3, **dadurch gekennzeichnet, dass** er darüber hinaus wenigstens ein Alkalimetall oder Erdalkalimetall umfasst.

5. Katalysator nach Anspruch 4, **dadurch gekennzeichnet, dass** das Atomverhältnis von Alkali- oder Erdalkalimetall zu Palladium bei 2 bis 20 liegt.

6. Katalysator nach einem der Ansprüche 4 und 5, **dadurch gekennzeichnet, dass** sein Gehalt an Alkali- oder Erdalkalimetall bei 0,05 bis 0,2 Gew.-% liegt.

7. Verfahren zur selektiven Hydrierung in gasförmiger Phase wenigstens eines actylenischen Kohlenwasserstoffs mit 2 oder 3 Kohlenstoffatomen zu entsprechendem ethylenischen Kohlenwasserstoff, **dadurch gekennzeichnet, dass** es das Leiten in gasförmiger Phase, einer Charge, die wenigstens einen acetylenischen Kohlenwasserstoff mit 2 oder 3 Kohlenstoffatomen in Anwesenheit von Wasserstoff über einen Katalysator in Form von Kugeln oder Extrudaten nach einem der Ansprüche 1 bis 6 umfaßt.

## Claims

1. A catalyst in the form of spherules or extrudates, comprising palladium, silver, and an alumina support, a proportion of at least 80% of the palladium and a proportion of at least 80% of the silver being present in a volume at the periphery of the catalyst delimited by a spherical or cylindrical surface with radius r₁ corresponding to the average radius of the spherules or extrudates of catalyst and a spherical or cylindrical surface with radius r₂ which is at least equal to 0.8 r₁, said catalyst being **characterized in that** the weight ratio of silver to palladium is from 0.4 to 3 and in that at least 30% of the metal particles on the alumina support contain both palladium and silver.

2. A catalyst according to claim 1, **characterized in that**:
• said alumina has a specific surface area of 5 to 60 m²/g;
• the palladium content is 0.01% to 0.5% by weight; and
• the silver content is 0.001% to 0.1% by weight.

3. A catalyst according to any one of claims 1 and 2, **characterized in that** the alumina has a specific surface area of 5 to 15 m²/g.

4. A catalyst according to any one of claims 1 to 3, **characterized in that** it also contains at least one alkali or alkaline-earth metal.

5. A catalyst according to claim 4, **characterized in that** the atomic ratio of alkali or alkaline-earth metal to palladium is 2 to 20.

6. A catalyst according any one of claims 4 and 5, **characterized in that** the alkali or alkaline-earth metal content is from 0.05% to 0.2% by weight.

7. A process for selective gas phase hydrogenation of at least one acetylenic hydrocarbon containing 2 or 3 carbon atoms to the corresponding ethylenic hydrocarbon, **characterized in that** it comprises passing a feed comprising at least one acetylenic hydrocarbons containing 2 or 3 carbon atoms in the gas phase in the presence of hydrogen over a catalyst which is in the form of spherules or extrudates according to any one of claims 1 to 6.
